(19) Europäisches Patentamt
European Patent Office
Office européen des brevets

(11) Publication number: **0 244 196 B1**

(12) **EUROPEAN PATENT SPECIFICATION**

(45) Date of publication of patent specification: **29.01.92**

(51) Int. Cl.⁵: **C07C 245/06**, G01N 33/84

(21) Application number: **87303728.7**

(22) Date of filing: **28.04.87**

The file contains technical information submitted after the application was filed and not included in this specification

(54) **Hydroxy-substituted cyanoformazans and their use in analytical compositions and methods.**

(30) Priority: **29.04.86 US 857001**

(43) Date of publication of application:
**04.11.87 Bulletin 87/45**

(45) Publication of the grant of the patent:
**29.01.92 Bulletin 92/05**

(84) Designated Contracting States:
**CH DE FR GB LI**

(56) References cited:
**EP-A- 0 108 924**
**GB-A- 1 491 608**

**CHEMICAL ABSTRACTS, vol. 98, no. 14, 4th April 1983, page 723, column 2 - page 724, column 1, abstract no. 118664w, Columbus, Ohio, US; Y. FENG et al.: "Studies on analytical characteristics of cyanoformazan derivative-surfactant systems", & HUAXUE SHIJI 1982, 4(4), 219-222, 230**

(73) Proprietor: **EASTMAN KODAK COMPANY (a New Jersey corporation)**
**343 State Street**
**Rochester New York 14650(US)**

(72) Inventor: **Babb, Bruce Edward, c/o EASTMAN KODAK COMPANY**
**Patent Department, 343 State Street**
**Rochester, NY 14650(US)**
Inventor: **Hilborn, David Alan, c/o EASTMAN KODAK COMPANY**
**Patent Department, 343 State Street**
**Rochester, NY 14650(US)**

(74) Representative: **Phillips, Margaret Dawn et al**
**Kodak Limited Patent Department Headstone Drive**
**Harrow, Middlesex HA1 4TY(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**CHEMICAL ABSTRACTS, vol. 92, no. 17, 28th April 1980, page 555, columns 1, 2, abstract no. 146397f, Columbus, Ohio, US; I.A. SHIKHOVA et al.: "Complexing of formazans with yttrium and cerium", & TR. INST. KHIM., URAL. NAUCHN. TSENTR, AKAD. NAUK SSSR 1978, 37, 63-69**

**CHEMICAL ABSTRACTS, vol. 84, no. 15, 12th April 1976, page 539, column 1, abstract no. 105165v, Columbus, Ohio, US; I.A. SHIKHOVA et al.: "Synthesis and acid-base properties of asymmetric cyanoformazans", & TR. INST. KHIM., URAL. NAUCHN. TSENTR, AKAD. NAUK SSSR 1974, 30, 40-46**

**CHEMICAL ABSTRACTS, vol. 88, no. 14, 3rd April 1978, page 69, column 2, abstract no. 91025t, Columbus, Ohio, US; M.I. ERMAKOVA et al.: "Synthesis of 1,5-diaryl-3-tetrazolylformazans", & KHIM. GETEROTSIKL. SOEDIN. 1977, (12), 1679-1681**

**CHEMICAL ABSTRACTS, vol. 99, no. 24, 12th December 1983, page 651, columns 1/2, abstract no. 205236x, Columbus, Ohio, US; R. YU et al.: "Catalytic-kinetic spectrophotometric determination of trace manganese(II) with 1,5-bis(2-hydroxy-5-chlorophenyl)-3-cyanoformazan", & HUAXUE XUEBAO 1983, 41(10), 960-965**

**J.A.Dean Handbook of organic chemistry (1987), section 7, 7-b**

**March, Advanced Organic Chemistry (1968), 238-241**

## Description

This invention relates to clinical chemistry. More specifically, it relates to novel cyanoformazan compounds and their use in analytical compositions and methods for the determination of magnesium ions.

Magnesium in its ionic form is essential to many physiological processes. It is one of the most abundant cations in the body and, next to potassium, it is the most prevalent intracellular ion. It plays a vital role in carbohydrate and lipid metabolism by serving as an activator of adenosine triphosphate (ATP) in the transfer of energy rich phosphate. It is also essential as an activating ion for many enzymes involved in lipid, carbohydrate and protein metabolism. In muscle tissue, magnesium has a significant influence on neuromuscular apparatus.

The amount of magnesium in the body is particularly significant. Decreased levels of magnesium in the body produce muscle irritability which, if not corrected, can result in involuntary muscle spasms and convulsions. On the other hand, increased levels of magnesium can result in a loss of deep tendon reflexes, a loss of touch, temperature and pain sensation, respiratory failure and cardiac arrest.

Therefore, it has been long recognized that for suitable diagnosis and treatment of various ailments, the accurate and rapid measurement of magnesium ions is important. In addition, it is also important in many environmental monitoring programs and manufacturing processes that magnesium be accurately measured.

Colorimetric methods are known for the determination of the concentration of magnesium ions in various fluids, for example groundwater, seawater, wastewater, manufacturing liquids and biological fluids. These methods usually involve adding a reagent to the fluid which forms a colored complex with any magnesium ions present. The complex absorbs electromagnetic radiation at a characteristic wavelength different from that of uncomplexed reagent.

Known methods for determining magnesium have various drawbacks. The fluids to be tested often contain various materials which interfere with the assay. For example, proteins and calcium ions present in fluids can also complex with magnesium complexing dyes, thereby causing an interference.

Hydroxy-substituted cyanoformazan derivatives have been used in the analysis of ions in fluids for some time. One compound specifically used in this manner is 1,3-bis(2-hydroxy-5-sulfophenyl)-3-cyanoformazan. While this compound has been found to successfully complex with chromium, copper, nickel and a variety of other transition metal ions, it does not selectively complex with magnesium ions at relatively low pH (that is, less than 10). Other similar cyanoformazans which complex aluminum ions at pH 4 are known in the art. None of these compounds are known to complex magnesium ions.

Cyanoformazan derivatives are described by Feng et al in the Chinese journal Chemical Reagents, 4(4), pp. 219-222 (1982). This reference describes an evaluation of the effect of surfactants on 1,5-bis(2-hydroxy-5-sulfophenyl)-3-cyanoformazan and 1,5-bis(2-hydroxy-5-chlorophenyl)-3-cyanoformazan. However, these compounds must be used at relatively high pH, that is, greater than 11, for greatest sensitivity for magnesium ions. At a pH below 11, their selectivity for magnesium is low. Further, the 5-sulfophenyl derivative exhibits high background in a magnesium assay. Also, the stability of the dyes decreases with increasing pH, that is, they tend to break down at higher pH and cannot be stored for an extended period of time.

Hence, there is a need in the art for compounds which have high sensitivity for magnesium ions at relatively low pH.

In accordance with the present invention there is provided a 1,5-bis(2-hydroxyphenyl)-3-cyanoformazan characterized wherein it is substituted in the 3-, 4- or 5-positions of either phenyl moiety with one or more halo, sulfamoyl or sulfoalkyl groups only such that the cumulative Hammett-sigma value of the substituents is greater than 0.35, and the cyanoformazan is capable of complexing with magnesium ions at a pH of from 8.5 to 10.5.

In accordance with the present invention there is also provided a composition for the determination of magnesium ions buffered to a pH of from 8.5 to 10.5 comprising a nonionic or anionic surfactant and a 1,5-bis(2-hydroxyphenyl)-3-cyanoformazan, the composition characterized wherein the cyanoformazan is substituted in the 3-, 4- or 5-positions of either phenyl moiety with one or more halo, sulfamoyl or sulfoalkyl groups only such that the cumulative Hammett-sigma value of the substituents is greater than 0.35, and the cyanoformazan is capable of complexing with magnesium ions at a pH of from 8.5 to 10.5.

In accordance with the present invention there is further provided a method for the determination of magnesium ions comprising the steps of:

A. contacting a sample of a liquid suspected of containing magnesium ions with a 1,5-bis(2-hydroxyphenyl)-3-cyanoformazan, and

B. detecting the color change resulting from the complexing of magnesium ions with the cyanoformazan,

the method characterized wherein it is carried out at a pH of from 8.5 to 10.5, and the cyanoformazan is

substituted in at least one of the 3-, 4- or 5-positions of either phenyl moiety with a substituent such that the cumulative Hammett-sigma value of said substituents is greater than 0.23, the substituent being a halo, sulfo, alkylsulfonyl, sulfamoyl, cyano, haloalkyl, carboxamide, carboxyalkyl or sulfoalkyl.

The present invention provides a number of advantages. Generally, it can be used to determine magnesium ions in various liquids at a pH of from 8.5 to 10.5. In this pH range, the cyanoformazan compounds have excellent stability and high selectivity for magnesium ions. It is preferable to carry out magnesium assays at a pH below 10.5 because at the higher pH, the assay is likely to have higher background from dye instability. Also, in the dry assays, it is difficult to control keeping when the element is designed for high pH assay.

With the novel compounds of this invention, which are preferred in the practice of the assay, the color change resulting from the presence of magnesium ions can be readily detected at longer wavelengths, that is, greater than 600 nm, thereby minimizing the background problem. It has also been observed that many of these compounds exhibit reduced affinity for complexation with protein molecules, thereby reducing the potential for protein interference.

The Figure is a plot of absorbance scans for a cyanoformazan dye alone and the dye complexed with magnesium ions as described in Example 10 below.

The compounds useful in the method of this invention are 1,5-bis(2-hydroxyphenyl)-3-cyanoformazans which are substituted in at least one of the 3-, 4- and 5-positions of either phenyl moiety with a substituent defined in claim 3 such that the cumulative Hammett-sigma value of the substituents is greater than 0.23 which is approximately the Hammett-sigma value for a single chloro substituent in either the 3- or 5-position. In a preferred embodiment using the novel compounds of this invention, the cumulative Hammett-sigma value is greater than 0.35. It is critical that the cyanoformazans described herein be capable of complexing with magnesium ions at a pH of from 8.5 to 10.5. Such complexing property can be readily evaluated by putting a given compound in a solution buffered to a pH of from 8.5 to 10.5, and observing whether or not a color change occurs when magnesium ions are added to the solution. If a color change occurs, complexation has taken place.

Hammett-sigma values ($\sigma$) are standard values used to predict the electron-withdrawing or electron-donating effect of substituents on phenyl rings. Such values can be calculated according to standard procedures described, for example in Steric Effects in Organic Chemistry, John Wiley & Sons, Inc., pp. 570-574 (1956) and Progress in Physical Organic Chemistry, Vol. 2, Interscience Publishers, pp. 333-339 (1964). Hammett-sigma values for some representative substituents are listed in the text by March, Advanced Organic Chemistry: Reactions, Mechanisms, and Structure, McGraw-Hill Book Company, New York, pp.238-241 (1968). The cumulative Hammett-sigma values shown herein are for substituents on the 3-, 4- and 5-positions only.

Representative substituents include halo (fluoro, chloro or bromo), sulfo, alkylsulfonyl (such as methylsulfonyl), sulfamoyl (that is, $-SO_2NR_1R_2$ wherein $R_1$ and $R_2$ are independently hydrogen, substituted or unsubstituted alkyl of 1 to 12 carbon atoms, for example, methyl, ethyl, isopropyl, benzyl, dodecyl or chloromethyl, cycloalkyl of 4 to 6 carbon atoms, for example, cyclobutyl or cyclohexyl as well as a chain of alkylene or cycloalkylene groups separated by oxy or thio linkages), cyano, substituted or unsubstituted haloalkyl (for example, mono-, di- or trihaloalkyl wherein the alkyl has from 1 to 12 carbon atoms, such as chloromethyl, dibromomethyl or 1,2-dichloroethyl), carboxamide, substituted or unsubstituted carboxyalkyl (wherein the alkyl has from 1 to 12 carbon atoms as defined above for $R_1$) and substituted or unsubstituted sulfoalkyl (wherein the alkyl has from 1 to 12 carbon atoms as defined above for $R_1$).

Particularly useful substituents include chloro, sulfamoyl and substituted or unsubstituted sulfoalkyl as defined above. It is also preferred that in the compounds of this invention one or more of the 3-, 4- and 5-positions of one phenyl ring of the compound have the same respective substituents as the corresponding positions of the other phenyl ring. Most preferably, the 3-and/or 5-positions are substituted.

Representative novel cyanoformazan derivatives of this invention include the following compounds. In a number of cases, there are given the cumulative Hammett-sigma ($\sigma$) values of the phenyl ring substituents other than the 2-hydroxy:

1,5-bis(2-hydroxy-3,5-dichlorophenyl)-3-cyanoformazan, $\sigma$ of +0.46,
1,5-bis(2-hydroxy-5-sulfamoylphenyl)-3-cyanoformazan, $\sigma$ of +0.57,
1,5-bis[2-hydroxy-5-(N-butylsulfamoyl)phenyl]-3-cyanoformazan, $\sigma$ of +0.42,
1,5-bis[2-hydroxy-5-(N-hexylsulfamoyl)phenyl]-3-cyanoformazan, $\sigma$ of +0.42,
1,5-bis[2-hydroxy-5-(N-octylsulfamoyl)phenyl]-3-cyanoformazan, $\sigma$ of +0.40,
1,5-bis[2-hydroxy-5-(N-dodecylsulfamoyl)phenyl]-3-cyanoformazan, $\sigma$ of +0.40,
1,5-bis[2-hydroxy-5-(N,N-diethylsulfamoyl)phenyl]-3-cyanoformazan, $\sigma$ of +0.40,
1,5-bis(2-hydroxy-5-cyanophenyl)-3-cyanoformazan, $\sigma$ of +0.66,

1,5-bis[2-hydroxy-3-chloro-5-(N-butylsulfamoyl)phenyl]-3-cyanoformazan, σ of +0.65,

1,5-bis(2-hydroxy-3-methylsulfonylphenyl)-3-cyanoformazan, σ of +0.72, and

1,5-bis(2-hydroxy-5-sulfomethylphenyl)-3-cyanoformazan.

The first compound in the above list is preferred in the practice of the assay of this invention.

A further cyanoformazan useful in the practice of this invention is:

1,5-bis(2-hydroxy-3-sulfo-5-chlorophenyl)-3-cyanoformazan, σ of +0.32.

The novel compounds of this invention can be prepared using standard starting materials and the following general procedure: (1) a 2-hydroxyaniline substituted with the appropriate substituent(s) in the 3-, 4- or 5-position is reacted with sodium nitrite in hydrochloric acid, and 92) the resulting diazooxide is reacted with cyanoacetic acid in an azo coupling reaction to provide the cyanoformazan derivative. Detailed preparations of several compounds are provided in Examples 1-9 below.

The cyanoformazan compounds described herein have varying degrees of solubility in aqueous solution depending upon the phenyl ring substituents they have. If they are water-soluble, they can be dissolved in water or a buffer to form an aqueous composition. Since many of the compounds have limited water solubility, a suitable anionic or nonionic surfactant is preferably used with them to promote solubility. Surfactants having a positive charge are not generally useful because they may cause precipitation of the cyanoformazan compound.

Suitable nonionic surfactants are too numerous to mention but examples of such include: alkylarylpolyethoxy alcohols, p-alkylaryloxypolyglycidols, and fluorocarbon surfactants.

A variety of anionic surfactants can also be used. Representative surfactants include sodium dodecyl sulfate, sodium octyl sulfate, and others known in the art.

Water-miscible organic solvents may also be included in the analytical composition in minor amounts to promote solubility of the cyanoformazan derivative. Such solvents include alcohols, N,N-dimethylformamide, dimethylsulfoxide, acetone or acetonitrile.

The composition of this invention is buffered to a pH of from 8.5 to 10.5 with one or more suitable buffers, for example, 2-(N-cyclohexylamino)ethane sulfonic acid, bicine, L-arginine, cyclohexylaminopropane sulfonic acid and others reported by Good et al in Biochem., 5, 467 (1966), and Anal. Biochem., 104, 300 (1980). Preferably, the composition is buffered to a pH of from 9 to 10.

Where a fluid to be assayed contains calcium ions in addition to magnesium ions, a suitable calcium ion chelating agent can be used to complex the calcium ions thereby preventing them from complexing with the cyanoformazan compound. A suitable chelating agent is 1,2-bis(o-aminophenoxy)ethane-N,N,N′N′-tetraacetic acid, and other compounds described by Tsien in Biochem., 19, pp. 2396-2404 (1980).

Generally, the cyanoformazan compound is present in the composition of the present invention at a concentration of at least 5, and preferably from 20 to 500, μmolar. The concentration of buffer to achieve the desired pH is within the skill of a worker in the art. The surfactant is generally present in an amount of at least 0.1, and preferably from 0.2 to 0.4, g/ml of solution.

The compositions of this invention can be used to advantage to assay a wide variety of aqueous liquids, for example, industrial, farm and residential wastewater, food and pharmaceutical processing solutions, food stuffs, groundwater, seawater or biological fluids. The invention is particularly useful for determining magnesium ions in various human and animal biological fluids, for example, whole blood, blood sera and plasma, urine, lymph fluid, spinal fluid, sputum, homogenized tissue or stool secretions. The practice of this invention is particularly important for the clinical assay of serum or urine.

A solution assay is generally carried out by contacting and mixing the composition described herein with a sample of fluid suspected of containing magnesium ions in a suitable container (for example, test tube, petri dish, beaker or cuvette). The resulting solution is mixed for a relatively short time at any suitable temperature (generally at least 25 °C). The solution is then evaluated by measuring the shift in spectral absorption caused by the complexation of the cyanoformazan derivative and magnesium ions at an appropriate wavelength using suitable colorimetric detection equipment. In many instances, the preferred cyanoformazan derivatives listed above form complexes with magnesium ions which can be detected at a wavelength greater than 600 nm whereas the derivative alone exhibits maximum absorption at a wavelength less than 600 nm.

The assay can also be carried out by contacting a porous absorbent material, for example a paper strip, containing the sample of fluid to be tested, with the composition of this invention. The magnesium ions in the fluid can migrate into and throughout the absorbent material and complex with the cyanoformazan to initiate the dye shift needed for magnesium ion determination.

Alternatively, the method of this invention can be practiced in a dry assay which is carried out with a dry analytical element. Such an element can be an absorbent carrier material, that is, a thin sheet or strip of self-supporting absorbent or bibulous material, such as filter paper or strips, which contains the novel

compound or a dried residue of the composition of this invention. Such elements are known in the art as test strips, diagnostic elements, dip sticks and diagnostic agents.

When employed in dry analytical elements, the composition of this invention can be incorporated into a suitable absorbent carrier material by imbibition or impregnation, or can be coated on a suitable material. Alternatively, it can be added to the element during an assay. Useful carrier materials are insoluble and maintain their structural integrity when exposed to water or physiological fluids. They can be prepared from paper, porous particulate structures, cellulose, porous polymeric films, glass fiber or woven and nonwoven fabrics (synthetic and nonsynthetic). Useful materials and procedures for making such elements are well known in the art as exemplified by U.S. Patents 3,092,465, 3,802,842, 3,915,647, 3,917,453, 3,936,357, 4,248,829, 4,255,384 and 4,270,920.

A dry assay can be practiced to particular advantage with an analytical element comprising a support having thereon at least one porous spreading zone as the absorbent carrier material. The spreading zone can be prepared from any suitable fibrous or non-fibrous material or mixtures of either or both as described in U. S. Patents 4,292,272, 3,992,158, 4,258,001 and 4,430,436, and Japanese Patent Publication 57(1982)-101760.

In the elements, the cyanoformazan derivative is generally present in a coverage of at least 0.1, and preferably from 0.2 to 1, $g/m^2$. Other reagents and materials (including buffer) are present in coverages within the skill of a worker in the art.

The assay using an element can be manual or automated. In general, in using the dry elements, magnesium ion determination is made by taking the element from a supply roll, chip packet or other source and physically contacting it with a sample (for example, 1 to 200 $\mu$l) of the liquid to be tested so that the sample is mixed with the cyanoformazan derivative in the element. Such contact can be accomplished in any suitable manner, for example, by dipping or immersing the element into the sample or, preferably, by spotting the element by hand or machine with one or more drops of the sample with a suitable dispensing means so that the liquid sample mixes with the reagents within the element.

After sample application, the element is exposed to any conditioning, such as incubation, heating or the like, that may be desirable to quicken or otherwise facilitate obtaining any test result.

Determination of magnesium ions is achieved by measuring the amount of dye shift resulting from complexation of the cyanoformazan derivative with the magnesium ions in the test sample using suitable equipment and procedures.

It is to be understood that not every cyanoformazan included in the scope of the method of this invention may be useful in both a solution and dry assay. For example, some compounds may be more useful in solution assays than dry assays. Other compounds may be more useful in dry assays than solution assays. Matching the compounds with a suitable assay is within the skill of a worker in the art.

Example 1: <u>Synthesis</u> <u>of</u> <u>1,5-bis(2-hydroxy-3,5-dichlorophenyl)-3-cyanoformazan</u>

The synthesis of 1,5-bis(2-hydroxy-3,5-dichlorophenyl)-3-cyanoformazan was carried out in the following manner.

Synthesis of 2-amino-4,6-dichlorophenol:

The starting material, 2,4-dichloro-6-nitrophenol (260 g, 1 mole, 20% in water), was mixed with methanol (2 liters) and platinum oxide catalyst, and the resulting slurry was reacted with hydrogen at 4.2 $kg/cm^2$ (60 psi) and room temperature.

The resulting solution was filtered to remove the catalyst and concentrated hydrochloric acid (150 ml) was added to the filtrate. The methanol was then removed by evaporation and the residual solid was redissolved in hot water (2 liters) with a little concentrated HCl added. The solution was filtered to remove dark, insoluble material and cooled to 30 °C. Dilute NaOH solution was added until the pH was about 6, and the mixture was chilled in an ice bath. The resulting white solid was filtered off and dried at room temperature under nitrogen. The compound yield was about 142 g (80% of theoretical) and it had a m.p. of 93-95 °C.

Synthesis of Cyanoformazan Derivative:

The product of the previous step (72 g, 0.4 mole) was dissolved in a solution of 50% NaOH (30 g) in water (500 ml). Sodium nitrite (30 g, 0.43 mole) was added to the mixture with stirring until the salt was dissolved. The resulting solution was dripped into a mixture of concentrated HCl (200 ml) and ice. During the addition, the temperature was kept below 5 °C by external cooling and occasional addition of ice. After the addition was complete, the slurry was stirred for 15 minutes further, and the resulting yellow, solid diazo oxide was filtered off and washed with water. Without drying this solid, it was redissolved in N,N-dimethylformamide (1500 ml) with slight warming.

The resulting diazo oxide solution was run into a solution of cyanoacetic acid (40 g, 0.47 mole) in water (500 ml) and 50% NaOH solution (200 ml). The reaction temperature was kept below 0 °C with cooling and addition of ice. The total volume completion was about 3 liters. The mixture became thick with precipitated dye and required stirring. After addition of the diazo oxide, the slurry was stirred in the ice/methanol bath for an hour, then warmed at 50 °C to dissolve the dye. Glacial acetic acid was added until the mixture was acidic, and filtration was carried out while the mixture was still warm. The resulting solid was washed with water and dried under vacuum at 80 °C under nitrogen.

The resulting dry, crude solid was added to N,N-dimethylformamide (700 ml) with heating, and glacial acetic acid (700 ml) was added to dissolve the dye under boiling conditions. After chilling and filtration, the resulting crystalline dye was washed with methanol and dried under vacuum to yield 65 g (77% theoretical) having a m.p. of 208-210 °C.

The product was evaluated by elemental analysis which confirmed the identity of the cyanoformazan derivative. Theoretical: C = 40.1, H = 1.7; N = 16.7. Found: C = 39.9, H = 1.8. N = 16.5. The derivative exhibited maximum absorption at 536 nm before complexation with magnesium ions and at 648 nm after complexation.

Examples 2-8: Preparation of Other Derivatives

A number of other cyanoformazan derivatives useful in the present invention were prepared in the following manner.

The starting materials, 3-amino-4-hydroxybenzene sulfonamides, were prepared using the standard synthetic method (Kermack et al, J. Chem. Soc., 608, 1939):

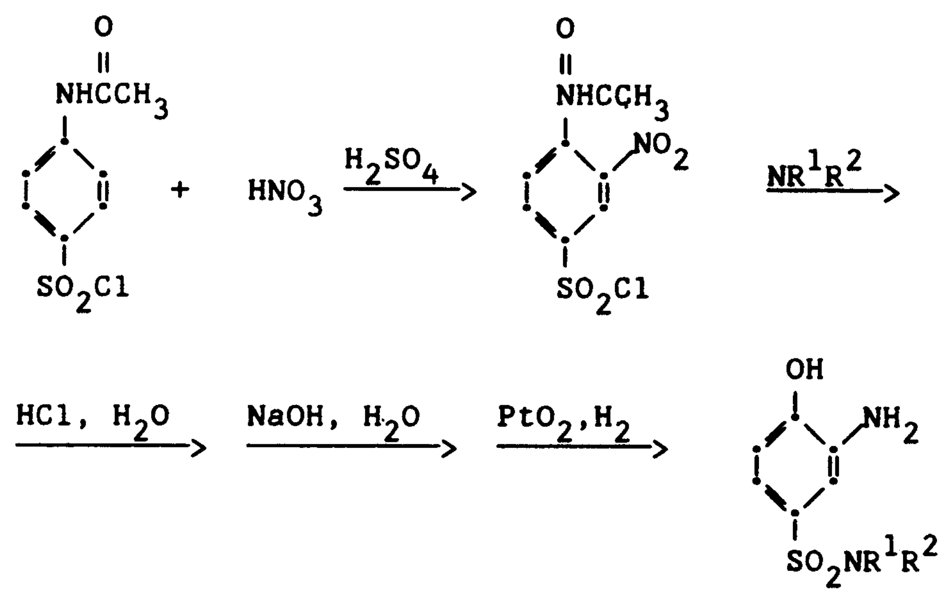


wherein $R^1$ and $R^2$ are defined above.

Other starting materials were prepared as follows:

A 3-amino-5-chloro-4-hydroxybenzene sulfonamide was prepared by direct chlorination of the 3-acetamido-4-hydroxybenzene sulfonamide followed by removal of the acetyl by acid hydrolysis. A cyanoaminophenol were prepared by catalytic reduction of the known nitrocyanophenol (J. Chem. Soc., 643, 1949). A 3-amino-4-hydroxyphenylmethyl sulfone was made by nitration of the known 4-acetamidobenzene sulfinic acid (Smiles et al, Org. Syn. coll. v-1,8).

The starting materials described above were used to prepare some of the cyanoformazan derivatives shown in Table I below according to the procedure described in Example 1 above. Other derivatives were similarly prepared using 2-hydroxyaniline as a starting material. Table I below lists the derivatives prepared as well as analytical and absorption data for each.

The dye-$Mg^{++}$ complex absorption in Table I were measured in a buffered composition containing 2-(N-cyclohexylamino)ethane sulfonic acid buffer (0.2 molar, pH 10), sodium chloride (0.15 molar) and TRITON X-100 surfactant (3%).

TABLE I

| Cyanoformazan Derivative | Absorption ($\lambda_{max}$, nm) Derivative | Derivative-$Mg^{++}$ Complex | Elemental Analysis Theoretical | Found |
|---|---|---|---|---|
| Example 2: 1,5-bis(2-hydroxy-5-cyanophenyl)-3-cyanoformazan | 528 | 620 | C=56.1, H=3.6 N=27.0 | C=56.2, H=3.9 N=27.6 |
| Example 3: 1,5-bis[2-hydroxy-5-(N,N-diethylsulfamoyl)-phenyl]-3-cyanoformazan | 522 | 620 | C=47.9, H=5.3 N=17.8 | C=47.1, H=5.1 N=17.4 |
| Example 4: 1,5-bis[2-hydroxy-5-(N-hexylsulfamoyl)phenyl]-3-cyanoformazan | 520 | 618 | C=51.4, H=6.1 N=16.1 | C=50.9, H=6.1 N=15.8 |
| Example 5: 1,5-bis[2-hydroxy-5-(N-dodecylsulfamoyl)phenyl]-3-cyanoformazan | 520 | 618 | C=58.8, H=7.9 N=12.6 | C=59.1, H=7.8 N=12.4 |

TABLE I (Cont'd.)

| Cyanoformazan Derivative | Absorption ($\lambda_{max}$, nm) Derivative | Derivative-$Mg^{++}$ Complex | Elemental Analysis Theoretical | Elemental Analysis Found |
|---|---|---|---|---|
| Example 6: 1,5-bis(2-hydroxy-5-methylsulfophenyl)-3-cyanoformazan | 536 | 614 | C=43.9, H=3.5 N=16.0 | C=43.3, H=3.6 N=15.7 |
| Example 7: 1,5-bis[2-hydroxy-3-chloro-5-(N-butylsulfamoyl)-phenyl]-3-cyanoformazan | 552 | 619 | C=42.6, H=4.4 N=15.8 | C=41.9, H=4.3 N=15.4 |
| Example 8: 1,5-bis[2-hydroxy-5-(N-octylsulfamoyl)phenyl]-3-cyanoformazan | 520 | 618 | C=54.3, H=6.8 N=14.8 | C=53.9, H=6.7 N=14.6 |

Example 9: Dye-Magnesium Ion Complexation

A solution of 1,5-bis(2-hydroxy-3,5-dichlorophenyl)-3-cyanoformazan (50 µmolar) was prepared in dimethylsulfoxide. A sample of this solution (20 µl) was added to 2 ml of buffer solution (pH 10) containing

0.2 molar 2-(N-cyclohexylamine)ethanesulfonic acid, 0.15 molar NaCl and 3% TRITON X-100 nonionic surfactant.

The absorbance of the resulting solution was measured with a standard spectrophotometer at room temperature. Magnesium ions (400 μmolar) were then added to the buffered solution and the absorbance was measured again. The two absorption scans are shown in the Figure. Complexation of the cyanoformazan derivative with magnesium ions causes a significant absorption shift.

A number of cyanoformazans outside the scope of this invention were prepared according to procedures similar to that in Example 1 above, and tested in an assay for magnesium ions as described above. The compounds tested were:

Control A: 1,5-bis(2-hydroxy-3-chlorophenyl)-3-cyanoformazan,
Control B: 1,5-bis(2-hydroxy-3,5,6-trichlorophenyl)-3-cyanoformazan,
Control C: 1,5-bis(2-hydroxy-3,5-dichloro-6-methylphenyl)-3-cyanoformazan.

None of these compounds were acceptable in an assay for magnesium ions. Control A did not successfully complex with magnesium ions at pH 10. It was determined that this derivative requires a pH higher than 10.5 for acceptable complexing with magnesium ions. Controls B and C, likewise, did not complex wit magnesium ions at pH 10, but required a pH greater than 11 for significant complexation.

Example 10: Stability of Dye-Mg Ion Complex

The example was carried out to determine the stability of the complex formed between magnesium ions and a cyanoformazan dye of this invention.

Increasing amounts of magnesium ions (up to 700 μmolar) were added to a buffered solution of 1,5-bis-(2-hydroxy-3,5-dichlorophenyl)-3-cyanoformazan prepared like that described in Example 10. The absorbance of the solution was measured at the spectra maximum after each increment of ions was added. The fraction of dye complexed was determined by measuring the difference in absorbance with and without ions at the absorbance maximum of the dye-Mg ion complex. This provided the determination of magnesium ions in each test. It was determined that the complex was highly stable as most of the dye remained complexed over the range of magnesium ion concentration.

TRITON X-100 referred to above, is a trade mark.

## Claims

**1.** A 1,5-bis(2-hydroxyphenyl)-3-cyanoformazan,

characterized wherein it is substituted in the 3-, 4- or 5-positions of either phenyl moiety with one or more halo, sulfamoyl or sulfoalkyl groups only such that the cumulative Hammett-sigma value of the substituents is greater than 0.35, and the cyanoformazan is capable of complexing with magnesium ions at a pH of from 8.5 to 10.5.

**2.** A composition for the determination of magnesium ions buffered to a pH of from 8.5 to 10.5 and comprising a nonionic or anionic surfactant and a 1,5-bis(2-hydroxyphenyl)-3-cyanoformazan,

the composition characterized wherein the cyanoformazan is substituted in the 3-, 4- or 5-positions of either phenyl moiety with one or more halo, sulfamoyl or sulfoalkyl groups only such that the cumulative Hammett-sigma value of the substituents is greater than 0.35, and the cyanoformazan is capable of complexing with magnesium ions at a pH of from 8.5 to 10.5.

**3.** A method for the determination of magnesium ions comprising the steps of:

A. contacting a sample of a liquid suspected of containing magnesium ions with a 1,5-bis(2-hydroxyphenyl)-3-cyanoformazan, and

B. detecting the color change resulting from the complexing of magnesium ions with the cyanoformazan,

the method characterized wherein it is carried out at a pH of from 8.5 to 10.5, and the cyanoformazan is substituted in at least one of the 3-, 4- or 5-positions of either phenyl moiety with a substituent such that the cumulative Hammett-sigma value of said substituents is greater than 0.23, the substituent being a halo, sulfo, alkylsulfonyl, sulfamoyl, cyano, haloalkyl, carboxamide, carboxyalkyl or sulfoalkyl.

**4.** The method as claimed in claim 3 carried out at a pH of from 9 to 10.

5. The method as claimed in claim 3 or 4 wherein the liquid is serum or urine.

6. The method as claimed in any one of claims 3 to 5 carried out in the presence of a nonionic or anionic surfactant in step A.

7. The invention as claimed in any one of claims 1 to 6 wherein in the cyanoformazan one or both of the 3- and 5-positions of one phenyl ring have the same respective substituents as the corresponding positions of the other phenyl ring.

8. The invention as claimed in any one of claims 1 to 7 wherein the cyanoformazan is selected from the group:
   1,5-bis(2-hydroxy-3,5-dichlorophenyl)-3-cyanoformazan,
   1,5-bis(2-hydroxy-5-sulfamoylphenyl)-3-cyanoformazan,
   1,5-bis[2-hydroxy-5-(N-butylsulfamoyl)phenyl]-3-cyanoformazan,
   1,5-bis[2-hydroxy-5-(N-hexylsulfamoyl)phenyl]-3-cyanoformazan,
   1,5-bis[2-hydroxy-5-(N-octylsulfamoyl)phenyl]-3-cyanoformazan,
   1,5-bis[2-hydroxy-5-(N-dodecylsulfamoyl)phenyl]-3-cyanoformazan,
   1,5-bis[2-hydroxy-5-(N,N-diethylsulfamoyl)phenyl]-3-cyanoformazan,
   1,5-bis(2-hydroxy-5-cyanophenyl)-3-cyanoformazan,
   1,5-bis[2-hydroxy-3-chloro-5-(N-butylsulfamoyl)phenyl]-3-cyanoformazan,
   1,5-bis(2-hydroxy-3-methylsulfonylphenyl)-3-cyanoformazan, and
   1,5-bis(2-hydroxy-5 sulfomethylphenyl)-3-cyanoformazan.

9. The invention as claimed in any one of claims 1 to 8 wherein the cyanoformazan is 1,5-bis(2-hydroxy-3,5-dichlorophenyl)-3-cyanoformazan.

**Revendications**

1. Composé 1,5-bis(2-hydrophényl)-3-cyano-formazan, caractérisé en ce qu'il est substitué en position 3-, 4- ou 5- sur l'un des groupes phényle par un ou plusieurs halogènes, des groupes sulfamyle ou sulfoalkyle de telle sorte que la valeur cumulée de la constante sigma de Hammett des substituants est supérieure à 0,35, et le cyanoformazan est capable de former un complexe avec les ions magnésium à un pH compris entre 8,5 et 10,5.

2. Composition pour la détermination des ions magnésium, tamponnée à un pH compris entre 8,5 et 10,5 et comprenant un tensioactif non-ionique ou anionique et un composé 1,5-bis(2-hydroxyphényl)-3-cyanoformazan, caractérisée en ce que le cyanoformazan est substitué en position 3-, 4- ou 5- sur l'un des groupes phényle par un ou plusieurs halogènes, groupes sulfamyle ou sulfoalkyle de telle sorte que la valeur cumulée de la constante sigma de Hammett des substituants est supérieure à 0,35, et le cyanoformazan est capable de former un complexe avec les ions magnésium à un pH compris entre 8,5 et 10,5.

3. Méthode pour la détermination des ions magnésium comprenant les étapes suivantes :
   A. mise en contact d'un échantillon de liquide pouvant contenir des ions magnésium avec un composé 1,5-bis (2-hydroxyphényl)-3-cyanoformazan, et
   B. détection d'un changement de couleur dû à la complexation des ions magnésium avec le cyanoformazan,

   caractérisée en ce que le pH est compris entre 8,5 et 10,5 et le cyanoformazan est substitué au moins en une des positions 3-, 4-, 5- de l'un des groupes phényle de telle sorte que la valeur cumulée de la constante sigma de Hammett des substituants est supérieure à 0,23, les substituants étant choisis parmi les atomes d'halogène, les groupes sulfo, alkylsulfonyle, sulfamyle, cyano, haloalkyle, carboxamide, carboxyalkyle ou sulfoalkyle.

4. Méthode selon la revendication 3 mise en oeuvre à un pH compris entre 9 et 10.

5. Méthode selon la revendication 3 ou 4 dans laquelle le liquide est le sérum ou l'urine.

6. Méthode selon l'une quelconque des revendications 1 à 5 mise en oeuvre en présence d'un tensioactif

non-ionique ou anionique au cours de l'étape A.

7. Invention selon l'une quelconque des revendications précédentes dans laquelle le cyanoformazan comprend sur les positions 3- et/ou les positions 5- des deux cycles phényles les mêmes substituants aux mêmes positions.

8. Invention selon l'une quelconque des revendications 1 à 7 dans laquelle le cyanoformazan est choisi parmi :
le 1,5-bis(2-hydroxy-3,5-dichlorophényl)-3-cyanoformazan,
le 1,5-bis(2-hydroxy-5-sulfamylphényl)-3-cyanoformazan,
le 1,5-bis[2-hydroxy-5-(N-butylsulfamyl)phényl]-3-cyanoformazan,
le 1,5-bis[2-hydroxy-5-(N-hexylsulfamyl)phényl]-3-cyanoformazan,
le 1,5-bis[2-hydroxy-5-(N-octylsulfamyl)phényl]-3-cyanoformazan,
le 1,5-bis[2-hydroxy-5-(N-dodécylsulfamyl)phényl]-3-cyanoformazan,
le 1,5-bis[2-hydroxy-5-(N,N-diéthylsulfamyl)-phényl]-3-cyanoformazan,
le 1,5-bis(2-hydroxy-5-cyanophényl)-3-cyanoformazan,
le 1,5-bis[2-hydroxy-3-chloro-5-(N-butylsulfamyl)-phényl]-3-cyanoformazan,
le 1,5-bis(2-hydroxy-3-méthylsulfonylphényl)-3-cyanoformazan et
le 1,5-bis(2-hydroxy-5-sulfométhylphényl)-3-cyanoformazan.

9. Invention selon l'une quelconque des revendications 1 à 8 dans laquelle le cyanoformazan est le 1,5-bis(2-hydroxy-3,5-dichlorophényl)-3-cyanoformazan.

**Patentansprüche**

1. Ein 1,5-Bis(2-hydroxyphenyl)-3-cyanoformazan,

dadurch gekennzeichnet, daß es in den 3-, 4- oder 5-Positionen beider Phenylreste durch ein oder mehrere Halo-, Sulfamoyl- oder Sulfoalkylgruppen substituiert ist, derart, daß der kumulative Hammett-Sigmawert der Substituenten größer als 0,35 ist und daß das Cyanoformazan Magnesiumionen bei einem pH-Wert von 8,5 bis 10,5 komplex zu binden vermag.

2. Zusammensetzung für die Bestimmung von Magnesiumionen, die auf einen pH-Wert von 8,5 bis 10,5 abgepuffert ist und ein nichtionogenes oder anionisches oberflächenaktives Mittel und ein 1,5-Bis(2-hydroxyphenyl)-3-cyanoformazan enthält, dadurch gekennzeichnet, daß das Cyanoformazan in den 3-, 4- oder 5-Positionen beider Phenylreste durch ein oder mehrere Halo-, Sulfamoyl- oder Sulfoalkylgruppen substituiert ist, derart, daß der kumulative Hammett-Sigmawert der Substituenten größer als 0,35 ist und daß das Cyanoformazan Magnesiumionen bei einem pH-Wert von 8,5 bis 10,5 komplex zu binden vermag.

3. Verfahren zur Bestimmung von Magnesiumionen mit den Stufen:

A. Kontaktieren einer Probe einer Flüssigkeit, von der angenommen wird, daß sie Magnesiumionen enthält, mit einem 1,5-Bis(2-hydroxyphenyl)-3-cyanoformazan, und
B. Bestimmung der Farbveränderung, die auf der Komplexbindung der Magnesiumionen durch das Cyanoformazan beruht,

dadurch gekennzeichnet, daß das Verfahren bei einem pH-Wert von 8,5 bis 10,5 durchgeführt wird und daß das Cyanoformazan in mindestens einer der 3-, 4- oder 5-Positionen beider Phenylreste mit einem Subtituenten substituiert ist, derart, daß der kumulative Hammett-Sigmawert der Substituenten größer als 0,23 ist, wobei der Substituent eine Halo-, Sulfo-, Alkylsulfonyl, Sulfamoyl-, Cyano-, Haloalkyl, Carboxamid-, Carboxyalkyl- oder Sulfoalkylgruppe ist.

4. Verfahren nach Anspruch 3, dadurch gekennzeichnet, daß es bei einem pH-Wert von 9 bis 10 durchgeführt wird.

5. Verfahren nach Anspruch 3 oder 4, dadurch gekennzeichnet, daß als Flüssigkeit Serum oder Urin verwendet wird.

6. Verfahren nach einem der Ansprüche 3 bis 5, dadurch gekennzeichnet, daß es in Gegenwart eines nichtionogenen oder anionischen Mittels in Stufe A durchgeführt wird.

7. Die Erfindung nach einem der Ansprüche 1 bis 6, bei der in dem Cyanoformazan eine oder beide der 3- und 5-Positionen eines Phenylringes die gleichen entsprechenden Substituenten aufweist wie die entsprechenden Positionen des anderen Phenylringes.

8. Die Erfindung nach einem der Ansprüche 1 bis 7, in der das Cyanoformazan ausgewählt ist aus der Gruppe von:

   1,5-Bis(2-hydroxy-3,5-dichlorophenyl)-3-cyanoformazan,

   1,5-Bis(2-hydroxy-5-sulfamoylphenyl)-3-cyanoformazan,

   1,5-Bis[2-hydroxy-5-(N-butylsulfamoyl)phenyl]-3-cyanoformazan,

   1,5-Bis[2-hydroxy-5-(N-hexylsulfamoyl)phenyl]-3-cyanoformazan,

   1,5-Bis[2-hydroxy-5-(N-octylsulfamoyl)phenyl]-3-cyanoformazan,

   1,5-Bis[2-hydroxy-5-(N-dodecylsulfamoyl)phenyl]-3-cyanoformazan,

   1,5-Bis[2-hydroxy-5-(N,N-diethylsulfamoyl)-phenyl]-3-cyanoformazan,

   1,5-Bis(2-hydroxy-5-cyanophenyl)-3-cyanoformazan,

   1,5-Bis[2-hydroxy-3-chloro-5-(N-butylsulfamoyl)-phenyl]-3-cyanoformazan,

   1,5-Bis(2-hydroxy-3-methylsulfonylphenyl)-3-cyanoformazan und

   1,5-Bis(2-hydroxy-5-sulfomethylphenyl)-3-cyanoformazan.

9. Die Erfindung nach einem der Ansprüche 1 bis 8, dadurch gekennzeichnet, daß das Cyanoformazan 1,5-Bis(2-hydroxy-3,5-dichlorophenyl)-3-cyanoformazan ist.

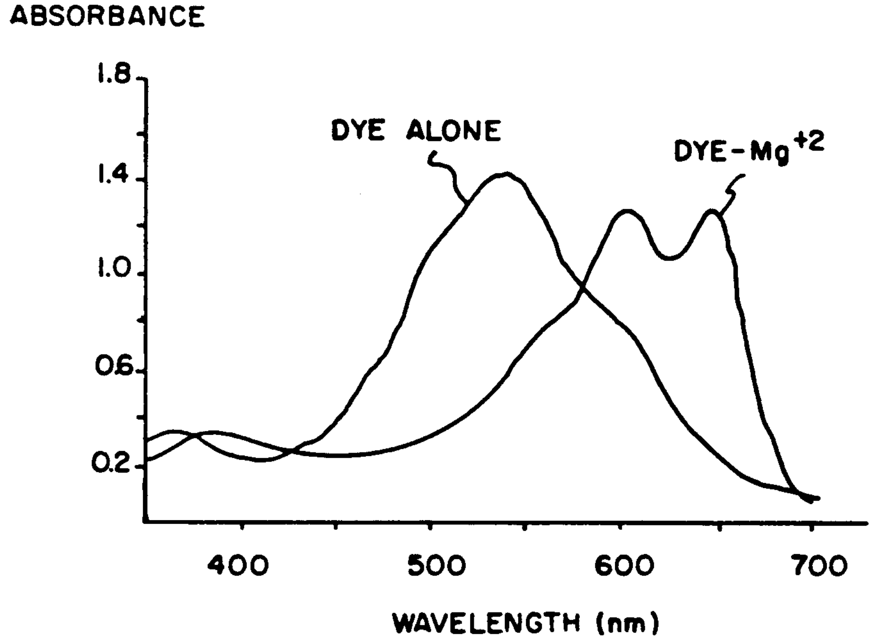
ABSORBANCE
1.8
1.4
1.0
0.6
0.2
DYE ALONE
DYE-Mg+2
400
500
600
700
WAVELENGTH (nm)

FIG. 1